# EUROPÄISCHE PATENTSCHRIFT

(11) **EP 1 326 115 B1**
(45) Veröffentlichungstag und Bekanntmachung des Hinweises auf die Patenterteilung: **21.02.2007**
(21) Anmeldenummer: 02100753.9
(22) Anmeldetag: 27.06.2002
(51) Int. Cl.: G02B 7/00, A61B 19/00, F16M 11/04

(54) **Stativ mit einer automatischen Balanciereinrichtung**
Stand with an automatic balancing device
Support avec dispositif automatique d'équilibrage

(30) Priorität: 04.01.2002 DE 20200079 U
(43) Veröffentlichungstag der Anmeldung: 09.07.2003
(73) Patentinhaber: Leica Microsystems (Schweiz) AG, 9435 Heerbrugg (CH)
(72) Erfinder: Metelski, Andrzej, 8590 Romanshorn (CH)
(74) Vertreter: Rosenich, Paul

(56) Entgegenhaltungen:
- DE-A- 4 320 443
- US-A- 5 047 641
- US-A- 6 045 104
- US-A- 6 129 319

## Beschreibung

Die Erfindung betrifft ein Stativ mit einer automatischen Balanciereinrichtung. Insbesondere im Bereich der Operationsmikroskopie werden mehr und mehr Stative eingesetzt, die das Gewicht des Mikroskops mittels automatischer Balanciereinrichtung ausbalancieren. Dabei werden in Abhängigkeit von Kräften oder Momenten, die durch eine Unbalance entstehen, Ausgleichsgewichte verschoben, so dass die Last (Mikroskop und dessen Zubehör) und die Ausgleichsgewichte in einen ausbalancierten Zustand kommen. Dieser Zustand ist dann erreicht, wenn ein Benutzer das Mikroskop am Stativ wie gewichtslos durch den Raum bewegen kann. In der Regel sind neben der automatischen Balanciereinrichtung auch Bremsen vorgesehen, die das Stativ in einer gewählten Stellung im Raum blockieren.

Herkömmliche Stative werden während des automatischen Balancierverfahrens mittels wenigstens einer der Bremsen eingebremst. Sodann wird die Unbalance gemessen (indem z.B. Knick-, Biege- oder Drehmomente oder Kräfte gemessen werden). Ein Rechner stellt anschließend - z.B. an Hand einer Tabelle - fest, ob das Stativ in Balance oder Unbalance ist. Befindet sich das Stativ in Unbalance, so wird ein Verstellbefehl für einen Verstellmotor generiert, der wenigstens eines der Ausgleichgewichte so verstellt, dass die Unbalance kleiner wird. Bei Bedarf wird dieser Vorgang wiederholt bis die Unbalance auf ein Minimum reduziert ist.

Dieses automatische Balancierverfahren ist, da die Messung der Unbalance im eingebremsten ruhenden Zustand der Last und des Ausgleichsgewichtes erfolgt, ein statisches Mess- und Regelverfahren. Der Erfinder nennt das bekannte Balancierverfahren daher statisches Balancierverfahren.

Aus der US 6,045,104 ist ein Stativ mit einem Mehrgelenk-Parallelogramm für ein Operationsmikroskop bekannt. Durch Veränderung des Mikroskopgewichts findet eine Verschiebung im Mehrgelenk-Parallelogramm statt und es wird ein Schalter für einen Elektromotors betätigt. Über den Elektromotor wird ein Ausgleichsgewicht verschoben. Es findet hier keine Messung mittels eines Sensors des tatsächlich auftretenden Biegemoments statt, sondern es wird lediglich mittels Schaltern eine Automatisierung der Bewegung des Ausgleichgewichts beschrieben.

Balancierverfahren werden beispielsweise beim OHS von Leica-Microsystems (Prospekt Nr. 10M15010EN) und bei dem Aufbau der DE-4320443-C2 angewendet.

Der Erfinder erkannte, dass die bekannten Systeme nachteilig sind in Bezug auf die folgenden Punkte:
a) Der Ablauf von Messung, Berechnung, Ansteuerung und Verstellung benötigt Zeit. Die Benutzer hätten jedoch gern ein sofortiges und vollständiges Ausbalancieren, ohne Zeit verstreichen lassen zu müssen.
b) Bei der schrittweisen Auflösung des Mess-, Ansteuer- und Verstellvorgangs, insbesondere bei der Wiederholung in mehreren Schritten kann im Stativ im Extremfall eine Schwingung erzeugt werden, die sich unangenehm bemerkbar macht und sogar auf die Messung einen negativen Einfluss haben kann.
c) Zur Vermeidung der Probleme gemäss b) muss mit dem Ausgleichsgewicht langsam gefahren werden, was die Problematik nach a) verstärkt.

Der Erfindung liegt somit die Aufgabe zugrunde, eine Vorrichtung zu schaffen, welche die angegebenen Nachteile vermeidet, indem der automatische Balanciervorgang beschleunigt werden soll, bzw. die dazu erforderliche Zeit reduziert wird und indem die schrittweise Auflösung des Balanciervorgangs möglichst in einen fliessenden Vorgang überführt werden soll.

Gelöst wird diese Aufgabe durch die Modifizierung der herkömmlichen Balanciereinrichtung zur Schaffung eines "dynamischen Balanciervorgangs". Dabei wird
i) auf die Messung eines statischen Ungleichgewichts-Zustandes verzichtet;
ii) die Verstellung des Ausgleichsgewichts während des Ausbalancierens ständig vorgenommen und
iii) während der Verstellung des Ausgleichsgewichts die sich ändernde Unbalance dynamisch gemessen.
iv) Ein Vergleichen mit Tabellen, Berechnen von Verstellbefehlen etc. kann entfallen. (Solche Vorgänge können jedoch auch beim dynamischen Balancieren vorgesehen sein, sie stellen jedoch nicht die Basis des erfindungsgemässen Systems dar, sie können u.U. lediglich zur Verfeinerung oder Verbesserung des Systems dienen. Entscheidend ist, dass bei der Erfindung während des Verstellens gemessen wird und nicht im statischen Zustand des Stativs.)

Der Aufbau des erfindungsgemässen neuen Stativs ist in Anspruch 1 angegeben.

Weitere Ausbildungen der Erfindung und Weiterentwicklungen sind in den abhängigen Schutzansprüchen angegeben.

Durch den vorgängig beschriebenen neuen Aufbau des Stativs mit der neuen Balancierung werden die nachstehenden Verbesserungen erreicht:
- Das vollständige Ausbalancieren eines Stativs erfolgt schneller;
- Das System neigt weniger zum Schwingen und die Messergebnisse sind exakter;
- Das neue System braucht weniger Rechnerleistung und ist deshalb auch weniger störanfällig.
- Durch den dynamischen Mess- und Verstellvorgang kann die Geschwindigkeit optimal an das Schwingungsverhalten des Gesamtsystems angepasst werden. Ein abrupter Stopp bei der Verstellbewegung findet nicht statt.

Im obigen und nachfolgenden Text wird zwar auf Stative für Operationsmikroskope Bezug genommen; die Erfindung ist jedoch nicht darauf eingeschränkt, sondern steht vielmehr auch anderen Benutzern optischer Geräte mit Stativen und automatischen Balanciervorgängen offen (z.B. Fernrohren, Projektoren, Video- und Photokameras, etc.).

Die Figuren werden zusammenhängend und übergreifend beschrieben. Gleiche Bezugszeichen bedeuten gleiche Bauteile, Bezugszeichen mit unterschiedlichen Indices geben funktionsgleiche Bauteile an. Tabellen oder Diagramme sind nicht zwingend skaliert und dargestellte Gegenstände sind nicht zwingend maßstabgerecht sondern nur symbolisch, da der Fachmann nach Kenntnis der Erfindung problemlos bestehende Aufbauten adaptieren kann, um zur erfindungsgemässen Lösung zu gelangen.

Es zeigen dabei:
Fig.1 einen symbolischen Gesamtaufbau des Stativaufbaus;
Fig.2 bis 4 symbolische Diagramme, die drei verschiedene schrittweise Balancierverläufe darstellen, welche beidseitig das Signal "Balance" (0-Signal) messen und das Ausgleichsgewicht auch über den ausbalancierten Zustand hinaus verschieben und
Fig.5 bis 7 symbolische Diagramme, die drei verschiedene schrittweise Balancierläufe darstellen, welche die 0-Signallinie nicht kreuzen, sondern sich grundsätzlich nur auf einer Seite des 0-Signals (Balancezustand) diesem BalanceZustand nähern.

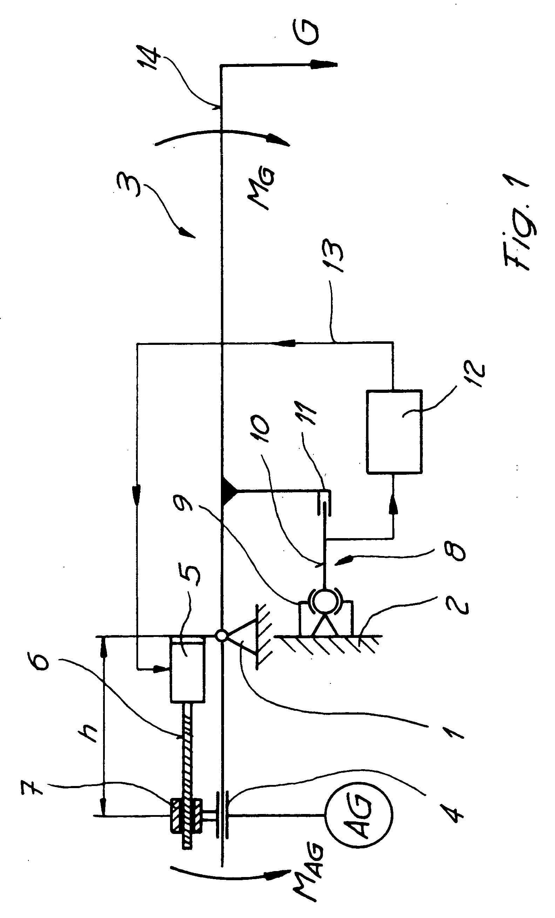

Wie der symbolische Aufbau gemäss Fig.1 zeigt, umfasst ein erfindungsgemässes ausbalancierbares Stativ ein Schwenklager 1, das - in Bezug auf einen nur angedeuteten Ständer 2 - grundsätzlich orts- und lagefest ist. Das Mikroskop bzw. die Last G hängt an einem Arm 14 eines Doppelarm-Trägers 3, der im Schwenklager 1 schwenkbar gelagert ist. Der Last G abgewandt auf dem anderen Arm (wirksamer Hebelarm h) des Trägers 3 befindet sich ein Ausgleichsgewicht AG, das zum Ausbalancieren verschiebbar ist. Es hängt an einem Schieber 4, der am Träger 3 verschiebbar gelagert ist. Ein Elektromotor 5, der am Träger 3 lagefest montiert ist, verstellt über eine Spindel 6, die mit einer Mutter 7 zusammenwirkt, den Abstand h zwischen Ausgleichsgewicht AG und Schwenklager 1. Die Mutter 7 ist mit dem Schieber 4 starr verbunden.
Das Stativ ist ausbalanciert, wenn das Kippmoment MG der Last gleich ist dem Ausgleichs-Kippmoment MAG. Während einer Verschiebebewegung des Ausgleichsgewichtes misst ein Sensor 8 die Grösse der jeweiligen Unbalance.

Der Sensor 8 ist wie folgt aufgebaut: Am Ständer 2 ist ein Messfühler 10 schwenkbar gelagert und - während des Messens - durch eine Bremse 9 gegenüber dem Ständer 2 gebremst. Ein gabelförmiger Mitnehmer 11, der mit dem Träger 3 fest verbunden ist, belastet den eingebremsten Messfühler 10 im Falle von Unbalance nach oben oder nach unten (positive oder negative Unbalance). Der Messfühler 10 umfasst einen nicht dargestellten Biegesensor, der mit einem Mikroprozessor 12 verbunden ist. Der Mikroprozessor 12 steuert über eine Steuerleitung 13 den Elektromotor 5.

Ebenso nicht dargestellt ist ein oberer und ein unterer Anschlag für den Messfühler 10, bei dem der Messfühler 10 infolge zu grosser Unbalance oben oder unten anliegt und keine weitere Messung bzw. Biegung zulässt. Das durch die Unbalance erzeugte überschüssige (zu grosse) Kippmoment MG oder Ausgleichs-Kippmoment (MG ist ungleich MAG) wird, im Anschlag anliegenden Zustand, über die Bremse direkt in den Ständer 2 geleitet und mechanisch aufgenommen. Nicht näher dargestellt ist ein Verriegelungssystem, das von Hand ver-, bzw. entriegelt werden kann, um den Träger 3 mit dem Ständer 2 zu arretieren - dies für den Fall grosser Gewichtsänderungen an der Last zur Schonung des Sensors 8. Durch diese Weiterentwicklung kann die Bremse 9 kleiner gebaut sein.

Der Sensor 8 liefert beim Anliegen am oberen Anschlag ein maximales positives Signal und beim Anliegen am unteren Anschlag ein maximales negatives Signal.

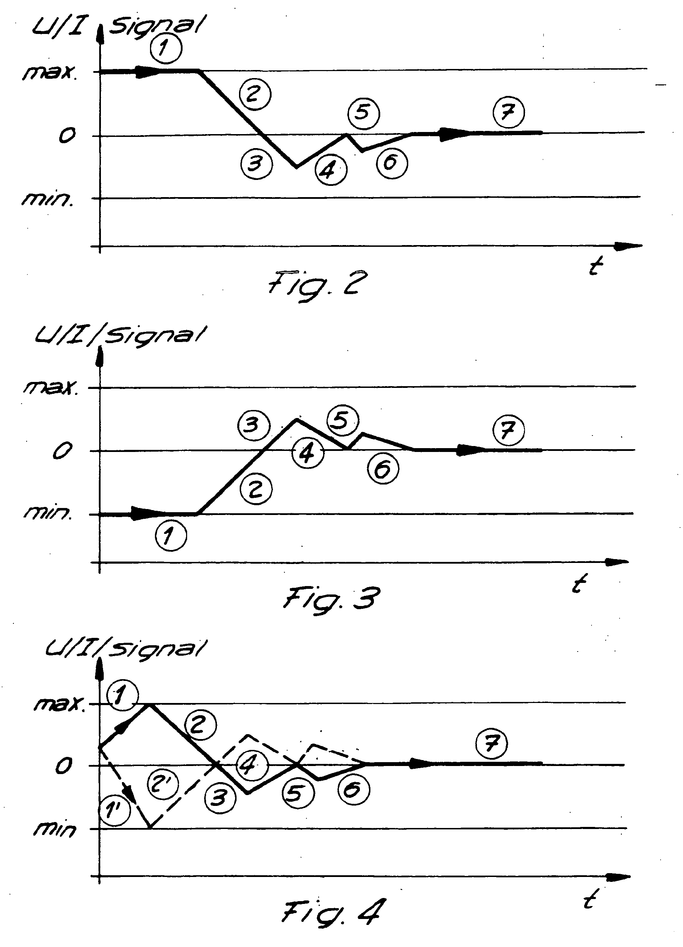

In Fig.2 sieht man einen solchen anliegenden Zustand bei positiver Unbalance und in Fig.3 einen solchen anliegenden Zustand bei negativer Unbalance, während Fig.4 eine Unbalance anzeigt, die innerhalb eines maximalen positiven oder negativen Unbalance-Signals liegt, bei dem der Messfühler 10 nicht an einem der Anschläge anliegt, die Unbalance somit nicht so gross ist.

### Funktionsweise beim erfindungsgemässen Ausbalancieren

Die eingekreisten Zahlen 1-7 in den Diagrammen der Fig.2-4 geben die Stufen des Balanciervorgangs an: In den Stufen 1-2 erfolgt ein Schnelllauf des Elektromotors 5, bei dem das Ausgleichsgewicht AG möglichst schnell verschoben wird - in die Negativrichtung, weil es am positiven Anschlag im Zustand gemäss Fig.2. anliegt und umgekehrt gemäss Fig.3. In einem Langsamlauf erfolgen sodann die weiteren Verstellungen des Ausgleichsgewichtes AG wieder zurück in die positive Richtung (Fig.2) - nach Überschreiten der 0-Signal-Linie (Signal bei balanciertem Zustand) bzw. umgekehrt in die negative Richtung (Fig.3).

Im Unterschied zum Bekannten, wird während des Verstellens des Ausgleichsgewichts AG gemessen, bzw. gemessen nachdem (und nicht zuvor) das Ausgleichsgewicht AG in Bewegung gesetzt wurde. Die Bewegungsrichtung des Ausgleichsgewichts AG im angeschlagenen Zustand ergibt sich aus dem tatsächlichen Anschlag beim positiven oder negativen Maximum. Weil die Messung auch im dynamischen Zustand (bei laufendem Ausgleichsgewicht AG) erfolgt, kann es bei diesem Ausführungsbeispiel zum Überschreiten der 0-Signal-Linie kommen, wie zwischen den Punkten 2 und 3 ersichtlich. Dabei ist die Verstellung des Ausgleichsgewichtes AG über den balancierten Zustand (0-Signal) hinaus vorgesehen, so dass ein Balancieren über der 0-Signal-Linie durchgeführt wird, wobei in diesem Ausführungsbeispiel beim Überschreiten der 0-Signal-Linie wenigstens ein Geschwindigkeitswechsel in der Verstellgeschwindigkeit (Verlangsamung) hervorgerufen wird. Gegebenenfalls erfolgt der Geschwindigkeitswechsel kontinuierlich und zwar angepasst an das Schwingungsverhalten des gesamten Systems. Ein solcher Geschwindigkeitswechsel kann jedes Mal beim Pendeln um die 0-Signal-Linie durchgeführt werden, was jedoch nicht zwingend ist.

Im Diagramm der Fig.4 ist der Ausgangspunkt des Balanciervorganges nicht bei einem Anschlag des Messfühlers 10 (am Rand oder ausserhalb des Messbereichs), sondern innerhalb des Messbereichs. Je nach Einstellung des Systems beginnt in diesem Fall die Verstellung des Ausgleichsgewichts AG durch ein Verschieben in die positive Richtung (fette Linie), so dass sich das Ungleichgewicht zunächst noch erhöht (1) und von dort, noch immer im Schnelllauf in die negative Richtung (2), bis zu einem Überschreiten der 0-Signal-Linie bei (3) und einer Verschiebeumkehr im Langsamlauf ab (3), die gegebenenfalls direkt bei der 0-Signal-Linie zum Stillstand kommt oder, wie dargestellt, im Langsamlauf nochmals weiterverschiebt, allerdings in einem kleineren Bereich, um eine Feinjustierung und ein genaues Erreichen der 0-Signal-Linie (genaue Balance) zu ermöglichen (5,6).

In Fig.4 ist gestrichelt noch eine alternative Verstellmöglichkeit angedeutet, bei der das Ausgleichsgewicht AG in die andere (negative) Richtung zu laufen beginnt. Bei dieser Variante ist es denkbar, dass schon beim 0-Durchgang ein Umkehrbefehl der Verschieberichtung ergeht, so dass u.U. der Balancezustand schneller erreicht wird als bei der erstgenannten Variante.

Die Anzahl der Verschiebebewegungen ist bei allen Diagrammen nur beispielhaft, so dass je nach Einstellung des Schnelllaufes, bzw. Langsamlaufes und je nach Trägheit des Verstellsystems mit dem Ausgleichgewicht AG und dem Elektromotor 5 mehr oder weniger Verschiebebewegungen möglich, bzw. notwendig sein können. Im Unterschied zum Bekannten kommt es also nicht darauf wesentlich an, wann wohin was verschoben wird, sondern dass während des Verschiebens die Balance gemessen und demzufolge die Verschiebebewegung beeinflusst, bzw. gesteuert wird.

Insofern könnte man den Gegenstand der Erfindung auch so darstellen, dass die Verstellbewegung des Ausgleichsgewichts AG von der Messung an sich unabhängig erfolgt, also nicht zuerst etwas gemessen und dann etwas verstellt wird, sondern die Verstellung läuft und in Abhängigkeit vom Verstellergebnis der weitere Verstellvorgang beeinflusst wird. Der Anfangsbefehl für die Laufrichtung im positiven oder negativen Max-Bereich (am Anschlag) kann durch die Messsoftware, insbesondere aber auch durch Endschalter bestimmt sein. Durch solche Endschalter kann somit verhindert werden, dass in einem unbestimmten Zustand der Balance (Messfühler am Anschlag) die Verstellbewegung in Richtung weiterer Unbalance läuft.

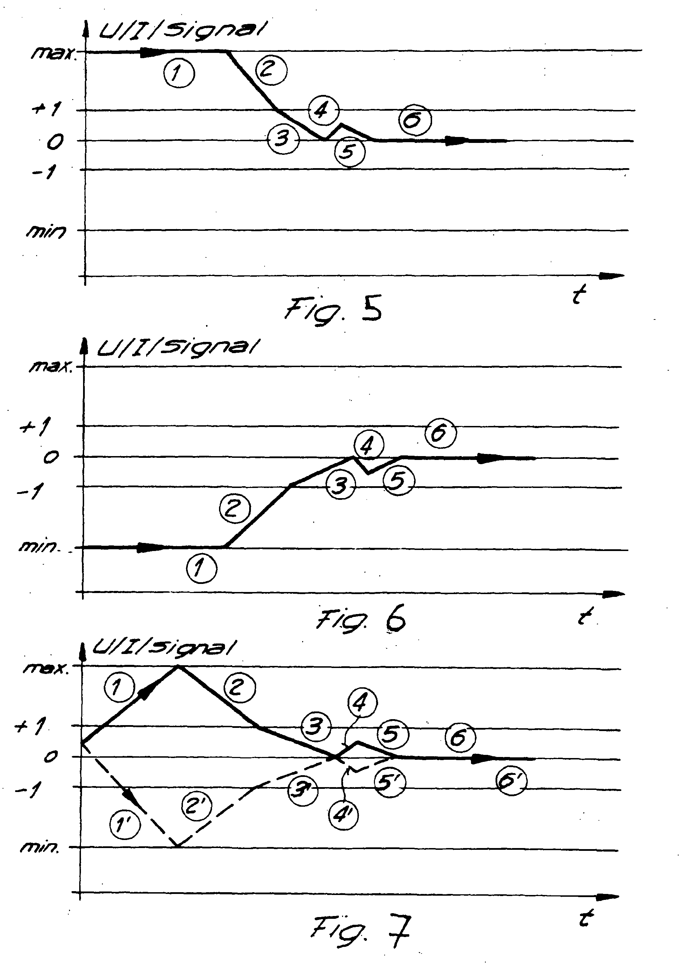

Aus den Fig.5 bis 7 sind erfindungsgemässe alternative Balancierverfahren symbolisch dargestellt, bei denen beim Verstellen des Ausgleichsgewichtes AG die 0-Linie grundsätzlich nicht überschritten wird. Die übrigen Merkmale des Balancierverfahrens sind gleich, so beispielsweise auch der Schnelllauf- und der Langsamlaufmodus. Dabei ist anzumerken, dass diese beiden Modi beispielhaft angegeben sind und das erfindungsgemässe Verfahren auch ohne unterschiedlicher Geschwindigkeiten oder mit einer grösseren Zahl von unterschiedlichen Geschwindigkeiten funktioniert.

Symbolisch und beispielhaft unterschiedlich zu den Verfahren nach den Diagrammen der Fig.2 bis 4 sind hier auch Messbereiche angegeben (zwischen -1 und +1), bei Erreichen derer automatisch in den Langsamlaufmodus geschaltet wird. Bei den Verfahren nach den Fig.5 bis 7 handelt es sich somit um Näherungsverfahren, getriggert nicht durch die 0-Linie sondern durch den -1 bzw. +1 Messbereich. In den Bereichen 1 und 2 erfolgt eine Schnelllaufverstellung des Ausgleichsgewichts AG und in den Bereichen 3 bis 6 eine Langsamlaufverstellung.

Fig.7 deutet darüber hinaus noch mittels gestrichelter Kurve eine Variante an, bei der der Balancierlauf gegen den negativen Anschlag erfolgt.

### Zusammenfassung gestützt auf das Symbolbild gemäss Fig.1:

Mit Hilfe des Elektromotors 5 wird (automatisch) der wirksame Hebelarm h durch Verschieben des Ausgleichgewichts AG verändert, unabhängig von der statischen Erfassung einer Unbalance. Während des Fahrens des Ausgleichsgewichts AG (Veränderung des Hebelarms h) wird durch einen Biegesensor o.dgl. (Wägezelle) am Messfühler 10 des Messsensors 8 der Gleichgewichtszustand (Balancezustand) dynamisch erfasst und gemäss den Diagrammen (Programme der Software im Mikroprozessor 12) der Fig.2 bis 7 ausgeregelt. Dabei können Schnell/Langsamläufe für den Elektromotor 5, Schwellenwerte im Messsignal, Anschläge und Endschalter vorgesehen sein.

### Bezugszeichenliste

- 1: Schwenklager
- 2: Ständer
- 3: Doppelarm-Träger
- 4: Schieber
- 5: Elektromotor
- 6: Spindel
- 7: Mutter
- 8: Messsensor
- 9: Bremse
- 10: Messfühler
- 11: Mitnehmer
- 12: Mikroprozessor
- 13: Steuerleitung
- 14: Lastarm
- h: wirksamer Hebelarm
- G: Last (Mikroskop)
- AG: Ausgleichsgewicht
- MG: Kippmoment der Last
- MAG: Kippmoment des Ausgleichsgewichts.

## Patentansprüche

1. Stativ mit einer automatischen Balanciereinrichtung, bestehend aus einem Ständer (2) und einem Träger (3), der mit diesem schwenkbar verbunden ist und an dessen Enden sich jeweils eine Last (G) und ein Ausgleichsgewicht (AG) befinden, welches an einem wirksamen Hebelarm (h) durch einen Elektromotor (5) verschiebbar ist und bei der mittels Messsensor (8) und Mikroprozessor (12) über Steuerleitungen (13) der Elektromotor (5) ansteuerbar ist, wobei der Mikroprozessor (12) ein Programm umfasst, **dadurch gekennzeichnet, dass** das Programm Programmschritte umfasst, bei denen bei laufender Verstellung des Ausgleichgewichtes (AG) zur Veränderung des wirksamen Hebelarms (h) die gemessene (Un-)Balance am Messsensor (8) festgestellt wird und der -somit dynamisch gemessene- Signalwert zur Ansteuerung des Elektromotors (5) verwendet wird, wobei durch diese Ansteuerung die laufende Verstellbewegung des Elektromotors (5) bei Bedarf beeinflusst wird, und dass der Messsensor (8) einen Messfühler (19) umfasst, der über eine Bremse (9) mit dem Ständer (2) während des Ausbalanciervorgangs gebremst verbindbar ist und mit einem mit demTräger (3) verbundenen Mitnehmer (11) die Bewegung des Trägers (3) erfasst.

2. Stativ nach Anspruch 1, **dadurch gekennzeichnet, dass** die Programmschritte Steuerbefehle umfassen, die die Verstellung des Ausgleichsgewichtes (AG) in unterschiedlichen Geschwindigkeiten, insbesondere in einem Schnelllauf und wenigstens einem Langsamlauf vornehmbar machen, wobei vorzugsweise durch den dynamischen Mess- und Verstellvorgang die Verstell-Geschwindigkeit optimal an das Schwingungsverhalten des Gesamtsystems angepasst ist und ein abrupter Stopp bei der Verstellbewegung nicht stattfindet und die Steuerbefehle derart sind, dass der Übergang zwischen unterschiedlichen Verstell-Geschwindigkeiten kontinuierlich erfolgt.

3. Stativ nach einem der vorhergehenden Ansprüchen, **dadurch gekennzeichnet, dass** die Programmschritte Steuerbefehle umfassen, die die Verstellung des Ausgleichsgewichtes (AG) über den balancierten Zustand (0-Signal) hinaus ermöglichen, so dass ein Balancieren über der 0-Signal-Linie durchführbar ist, wobei vorzugsweise beim Überschreiten der 0-Signal-Linie ein Geschwindigkeitswechsel in der Verstellgeschwindigkeit hervorrufbar ist.

4. Stativ nach einem der vorhergehenden Ansprüche, **dadurch gekennzeichnet, dass** ein oberer und ein unterer Anschlag vorhanden ist, an dem im Betriebszustand bei entsprechender positiver oder negativer Unbalance der Messsensor (8) bzw. sein Messfühler (10) anliegt und eine weiter differenzierte Messung unterbunden ist., wobei im Bereich der Anschläge Endschalter vorgesehen sind, die mit dem Mikroprozessor (12) verbunden sind und zur Laufrichtungsansteuerung des Elektromotors (5) bzw. des Ausgleichsgewichtes (AG) dienen.

5. Stativ nach einem der vorhergehenden Ansprüche, **dadurch gekennzeichnet, dass** der Lastarm und der Hebelarm (h) an einem Doppelarm-Träger (3) ausgebildet sind, der an einem Schwenklager (1) an dem Ständer (2) schwenkbar gelagert ist.

6. Stativ nach einem der vorhergehenden Ansprüche, **dadurch gekennzeichnet, dass** der Elektromotor (5) eine Spindel (6) umfasst, die mit einer Mutter (7) am Ausgleichsgewicht (AG) kooperiert.

7. Stativ nach einem der vorhergehenden Ansprüche, **dadurch gekennzeichnet, dass** der Elektromotor mit einer Spindel-Kugelumlaufmuttereinheit verbunden ist, die in einen Schieber (4) am Ausgleichsgewicht eingreift.

8. Stativ nach einem der vorhergehenden Ansprüche, **dadurch gekennzeichnet, dass** wenigstens der Lastarm und/oder der wirksame Hebelarm (h) als Parallelogrammträger ausgebildet ist/sind.

9. Stativ nach einem der vorhergehenden Ansprüche, **dadurch gekennzeichnet, dass** das Ausgleichsgewicht (AG) über einen Krafteinleitungsschieber mit dem wirksamen Hebelarm verbunden und selbst immer in einem konstanten Abstand zum Ständer (2) gehalten ist.

10. Stativ nach einem der vorhergehenden Ansprüche, **dadurch gekennzeichnet, dass** ein Verriegelungssystem vorgesehen, das von Hand ver- bzw. entriegelbar ist, um den Träger (3) mit dem Ständer (2) zu arretieren.

## Claims

1. Stand having an automatic balancing device, comprising a stand column (2) and a support (3) which is connected to the former such that it can pivot, with a load (G) and a counterbalancing weight (AG) at the ends of the support (3), which counterbalancing weight (AG) can be moved by an electric motor (5) on an acting lever arm (h), in which case the electric motor (5) can be driven by means of a measurement sensor (8) and a microprocessor (12) via control lines (13), with the microprocessor having a program, **characterized in that** the program comprises program steps in which, during movement of the counterbalancing weight (AG) in order to vary the effective lever arm (h), the measured (un)balance is determined at the measurement sensor (8) and the signal value (which is measured dynamically in this way) is used to drive the electric motor (5), wherein the continuous adjusting movement of the electric motor (5) is influenced by this drive as required, and **in that** the measurement sensor (8) has a measurement sensor (19) which can be connected, via a brake (9), to the stand column (2) during the balancing process in a braked manner and detects the movement of the support (3) using a driver (11) connected to the support (3).

2. Stand according to Claim 1, **characterized in that** the program steps comprise control commands which allow the counterbalancing weight (AG) to be moved at different speeds, in particular a fast mode and at least one slow mode, wherein the movement speed, on account of the dynamic measurement and movement process, is preferably optimally matched to the oscillatory behaviour of the entire system and no abrupt stop occurs during the movement, and the control commands are such that the transition between different movement speeds takes place continuously.

3. Stand according to one of the preceding claims, **characterized in that** the program steps comprise control commands which allow the movement of the counterbalancing weight (AG) beyond the balanced state (0 signal) such that balancing is possible via the 0 signal line, and it is preferably possible to cause a change in the movement speed when the 0 signal line is crossed.

4. Stand according to one of the preceding claims, **characterized in that** an upper and a lower stop are provided, which the measurement sensor (8) and its measurement sensor (10) restore in the operating state with corresponding positive or negative unbalance, with any further differential measurement being prevented, with limit switches being provided in the region of the stops, which limit switches are connected to the microprocessor (12) and are used to control the movement direction of the electric motor (5) and the counterbalancing weight (AG).

5. Stand according to one of the preceding claims, **characterized in that** the load arm and the lever arm (h) are formed on a double-arm support (3) which is mounted on a pivoting bearing (1) on the stand (2) such that it can pivot.

6. Stand according to one of the preceding claims, **characterized in that** the electric motor (5) has a spindle which interacts with a nut (7) on the counterbalancing weight (AG).

7. Stand according to one of the preceding claims, **characterized in that** the electric motor is connected to a spindle ball thread nut unit which engages in a slide (4) on the counterbalancing weight.

8. Stand according to one of the preceding claims, **characterized in that** at least the load arm and/or the effective lever arm (h) are/is in the form of a parallelogram support.

9. Stand according to one of the preceding claims, **characterized in that** the counterbalancing weight (AG) is connected to the effective lever arm via a force introduction slide, and is itself always held at a constant distance from the stand (2).

10. Stand according to one of the preceding claims, **characterized in that** a locking system is provided, which can be manually locked and unlocked in order to lock the support (3) to the stand (2).

## Revendications

1. Pied muni d'un dispositif d'équilibrage automatique, composé d'un montant (2) et d'un élément porteur (3) qui est relié avec lui de manière à pouvoir pivoter et aux extrémités duquel se trouvent respectivement une charge (G) et un contrepoids (AG), lequel peut être positionné sur un bras de levier efficace (h) par un moteur électrique (5) et avec lequel le moteur électrique (5) peut être commandé par le biais de lignes de commande (13) au moyen d'un capteur de mesure (8) et d'un microprocesseur (12), le microprocesseur (12) comprenant un programme, **caractérisé en ce que** le programme comprend des sections de programme avec lesquelles le (dés)équilibre au niveau du capteur de mesure (8) est déterminé lors d'un positionnement continu du contrepoids (AG) en vue de modifier le bras de levier efficace (h) et la valeur du signal, ainsi mesurée de manière dynamique, est utilisée pour commander le moteur électrique (5), cette commande influençant le mouvement de positionnement continu du moteur électrique (5) en cas de besoin et **en ce que** le capteur de mesure (8) comprend une sonde de mesure (19) qui peut être reliée de manière freinée par le biais d'un frein (9) au montant (2) pendant l'opération d'équilibrage et acquiert le mouvement de l'élément porteur (3) avec un élément d'entraînement (11) relié à l'élément porteur (3).

2. Pied selon la revendication 1, **caractérisé en ce que** les sections de programme comprennent des instructions de commande qui permettent de prévoir le positionnement du contrepoids (AG) à différentes vitesses, notamment à une vitesse rapide et au moins à une vitesse lente, la vitesse de positionnement étant adaptée de manière optimale au comportement oscillant de l'ensemble du système de préférence par le biais de l'opération de mesure et de positionnement dynamique et un arrêt brutal n'ayant pas lieu lors du mouvement de positionnement et les instructions de commande étant telles que la transition entre les différentes vitesses de positionnement s'effectue continuellement.

3. Pied selon l'une des revendications précédentes, **caractérisé en ce que** les sections de programme comprennent des instructions de commande qui permettent le positionnement du contrepoids (AG) au-delà de l'état d'équilibre (signal 0) de manière à pouvoir effectuer un mouvement de balancier au-delà de la ligne du signal 0, un changement de vitesse de positionnement pouvant de préférence être invoqué en cas de dépassement de la ligne du signal 0.

4. Pied selon l'une des revendications précédentes, **caractérisé en ce qu'**il existe une butée supérieure et inférieure au niveau desquelles se trouve le capteur de mesure (8) ou sa sonde de mesure (10) en situation de fonctionnement avec un déséquilibre positif ou négatif correspondant et une autre mesure différentielle est inhibée, des fins de course étant prévues dans la zone des butées, lesquelles sont reliées au microprocesseur (12) et servent à la commande du sens de rotation du moteur électrique (5) ou du sens de déplacement du contrepoids (AG).

5. Pied selon l'une des revendications précédentes, **caractérisé en ce que** le bras de charge et le bras de levier (h) sont formés sur un élément porteur à double bras (3) qui est monté de manière à pouvoir pivoter sur un palier de pivotement (1) sur le montant (2).

6. Pied selon l'une des revendications précédentes, **caractérisé en ce que** le moteur électrique (5) comprend une broche (6) qui agit conjointement à un écrou (7) sur le contrepoids (AG).

7. Pied selon l'une des revendications précédentes, **caractérisé en ce que** le moteur électrique est relié à une unité à broche de recirculation de billes qui vient en prise avec un coulisseau (4) sur le contrepoids.

8. Pied selon l'une des revendications précédentes, **caractérisé en ce qu'**au moins le bras de charge et/ou le bras de levier efficace (h) est/sont réalisé(s) sous la forme d'un élément porteur à parallélogramme.

9. Pied selon l'une des revendications précédentes, **caractérisé en ce que** le contrepoids (AG) est relié au bras de levier efficace par le biais d'un coulisseau d'introduction des forces et il est lui-même maintenu à un écart toujours constant par rapport au montant (2).

10. Pied selon l'une des revendications précédentes, **caractérisé en ce qu'**il est prévu un système de verrouillage qui est verrouillable ou déverrouillable manuellement pour bloquer l'élément porteur (3) avec le montant (2).
